# EUROPEAN PATENT APPLICATION

(11) **EP 4 653 546 A1**
(43) Date of publication of application: **26.11.2025**
(21) Application number: 24744891.3
(22) Date of filing: 17.01.2024
(51) Int. Cl.: C12Q 1/6869, C12Q 1/6883, C12N 9/22, C12N 9/78, C12N 15/113

(54) **CELL-FREE TEST METHOD FOR MEASURING BASE EDITING ABILITY OF BASE EDITOR**

(30) Priority: 18.01.2023 KR 20230007674
(71) Applicant: Edgene, Inc., Incheon-si 21990 (KR)
(72) Inventor: KIM, Jin Soo, Seoul-si 08505 (KR); LEE, Seong Hyun, Seoul-si 08505 (KR)
(74) Representative: Berggren Oy
(86) International application number: PCT/KR2024/000873
(87) International publication number: WO 2024/155111

(57) **Abstract**

The present invention relates to a method for testing genome editing in a cell-free, in vitro system and, in particular, to a method for identifying and quantifying target DNA mutations by a base editor without using eukaryotic cells and without separate complicated experimental procedures. By using the present invention, target DNA mutations can be identified and quantified by a base editor without using cells and without separate complicated experimental procedures. In addition, a base editor having the ability to edit target DNA mutations in actual cells can be screened through a simple cell-free test method.

## Description

### [Technical Field]

The present invention relates to a method for assaying genome editing in a cell-free, in vitro system and, more specifically, to a method for identifying and quantifying target DNA mutations using a base editor, without using cells and without requiring additional complex experimental procedures. The target DNA may be amplified from cells and used, but more advantageously, the target DNA may be used by mimicking mutant DNA of an organism (for example, a patient with a genetic disease) that requires base editing, through double-stranded DNA synthesis. By measuring DNA editing efficiency without the use of cell lines or model animals carrying specific mutations, the method may serve as a primary assay for evaluating the base-editing activity of various base editors, including adenine base editor, cytosine base editor, DddA-derived cytosine base editor (DdCBE), TALE-linked deaminase (TALED), zinc finger deaminase (ZFD), and the CRISPR system.

### [Background Art]

There are various proteins used as tools in genome editing. For example, base editing may employ a fusion protein in which a base-converting enzyme, such as a deaminase, is linked to nickase Cas9 (nCas9) or catalytically inactive Cas9 (dCas9), to induce single-base substitutions, such as A-to-G (adenine base editor) or C-to-T (cytosine base editor), while avoiding double-strand breaks (DSBs). Prime editing may be used to induce various programmable mutations, including nucleotide substitutions, small insertions, or deletions (indels), at specific positions in nuclear DNA, by using a fusion protein in which reverse transcriptase is linked to nCas9 and a prime editing guide RNA (pegRNA) comprising an RNA template. In addition, DdCBE and TALED technologies may induce single-base substitutions from cytosine to thymine or from adenine to guanine not only in nuclear DNA but also in organelle DNA.

### [Disclosure]

### [Technical Problem]

While it is relatively easy to apply genome editing to endogenous genes of an organism and evaluate the efficiency thereof because cell lines possessing normal genes may be used, conducting experiments targeting DNA having mutations is challenging. Although cells may be isolated from an animal model or patient with a desired mutation and may be directly tested in primary cells or used to generate cell lines for experiments, cell maintenance is difficult and a relatively long period of time (e.g., 3 to 4 months) is required. Accordingly, the purpose of the present invention is to provide a method for assaying genome editing in a cell-free, in vitro system, which is a method for identifying and quantifying target DNA mutations using a base editor, without using cells and without requiring additional complex procedures. The present invention further provides a method for screening a base editor capable of correcting a target DNA mutation in actual cells using such a simplified cell-free test method.

### [Technical Solution]

A cell-free test method according to the present invention involves contacting an isolated double-stranded DNA, which is an editing target, with a base-editing protein in a cell-free environment to obtain a base-edited DNA, and sequencing the obtained base-edited DNA to measure base editing activity. Here, the base-editing protein may be synthesized as a base-editing protein by mixing a polynucleotide (including DNA or mRNA) encoding the base-editing protein, or a vector comprising the same, with the target DNA in a single reaction tube (one pot), and may be used in the DNA base-editing reaction without a separate protein purification process, or may be used by being mixed with the target DNA in an expressed and purified form. The target DNA may be nuclear DNA, mitochondrial DNA, or chloroplast DNA, and may be extracted and amplified or synthesized from animal or plant cells for use. In this way, it has been confirmed for the first time that base editing in a cell-free environment, without using cells, exhibits substantially the same editing activity as when editing is performed in cells. Accordingly, it has become possible to measure the base-editing activity of a base editor by identifying target DNA mutations and measuring base-editing efficiency in a cell-free environment, without the need for complex experiments involving cells. Moreover, the present invention enables screening of base editors capable of correcting target DNA mutations in actual cells using only a simple cell-free test method.

### [Description of Drawings]

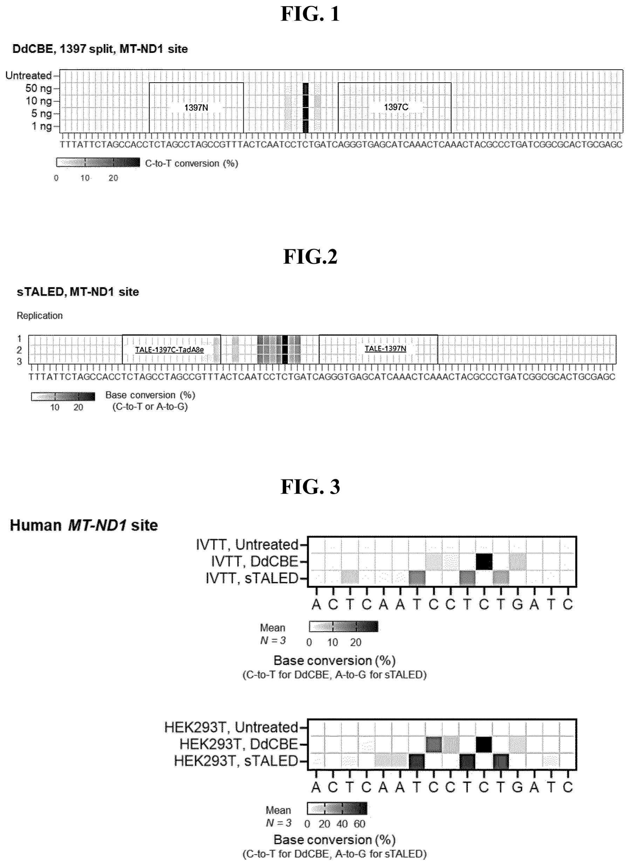

FIG. 1 shows the result of base editing of the ND1 gene region of human mitochondria using DdCBE (DddA-derived cytosine base editor) in a cell-free environment according to the present invention. 1397N represents the N-terminal G1397 DddA tox split, and 1397C represents the C-terminal G1397 DddA tox split. The boxed portion on the left side represents the DNA sequence recognized by the fusion protein comprising 1397N (left DdCBE), and the boxed portion on the right side represents the DNA sequence recognized by the fusion protein comprising 1397C (right DdCBE). The degree of intensity indicates the base-editing efficiency. A guanine (G) base position marked as having been base-edited indicates that the cytosine (C) base that forms the base pair at the corresponding position has been edited.
FIG. 2 shows the result of base editing targeting the ND1 gene region of human mitochondria using TALE (TALE-linked deaminase) in a cell-free environment according to the present invention. 1397N represents the N-terminal G1397 DddA tox split, and 1397C represents the C-terminal G1397 DddA tox split. The boxed portion on the left side represents the DNA sequence recognized by the fusion protein (left split TALED (sTALED)) comprising TALE proteins linked to 1397C and TadA8e, and the boxed portion on the right side represents the DNA sequence recognized by the fusion protein (right split TALED (sTALED)) comprising TALE proteins linked to 1397N. The degree of intensity indicates the base-editing efficiency. A thymine (T) base position marked as having been base-edited indicates that the adenine (A) base that forms the base pair at the corresponding position has been edited. The editing of the cytosine (C) base is the result of C-to-T editing caused by DddA tox included in TALED, and the fact that the guanine (G) base position is also marked as having been edited indicates that the cytosine (C) base that forms the base pair at the corresponding position has been edited.
FIG. 3 shows a comparison between the result of C-to-T editing using DdCBE and A-to-G editing using TALED for the ND1 gene region of human mitochondria in a cell-free environment according to the present invention, and the result of C-to-T editing using the same DdCBE and A-to-G editing using the same TALED in HEK293T cells. IVTT indicates that base editing was performed using a cell-free test method according to the present invention, and HEK293T indicates that base editing was performed using HEK293T cells.

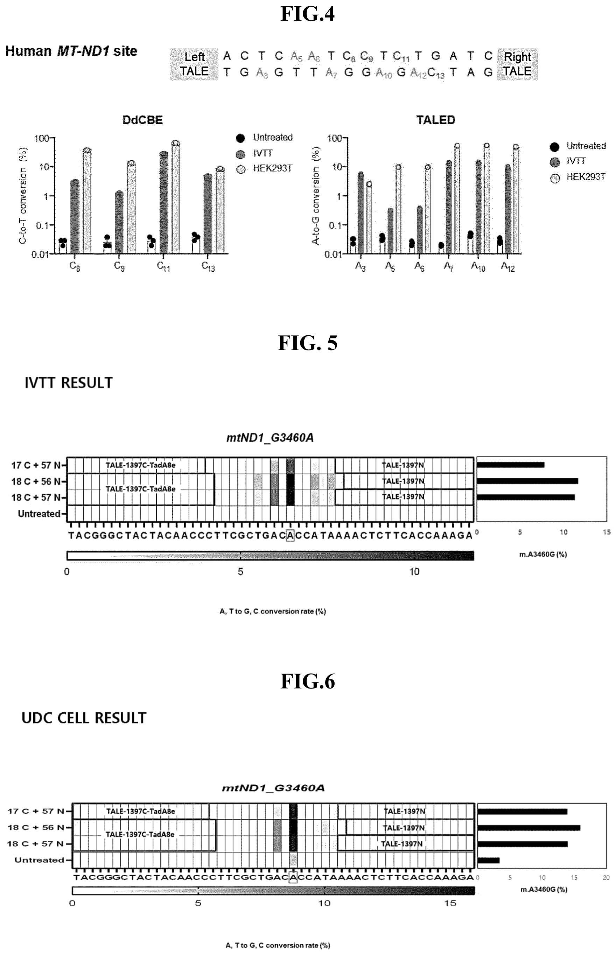

FIG. 4 is a bar graph comparing the C-to-T base-editing efficiency obtained using DdCBE and the A-to-G base-editing efficiency obtained using TALED for the ND1 gene region of human mitochondria in a cell-free environment according to the present invention, and the C-to-T base-editing efficiency obtained by applying the same DdCBE to HEK293T cells and the A-to-G base-editing efficiency obtained by applying the same TALED.
FIG. 5 shows the result of A-to-G base editing of the G3460A mutation (a mutation in which the 3460th base of the mitochondrion is A instead of G) in the ND1 gene of the mitochondria of a patient with Leber's hereditary optic neuropathy (LHON) using TALED in a cell-free environment according to the present invention. m.A3460G denotes that A-to-G editing is performed for the G3460A mutation. 1397N represents the N-terminal G1397 DddA tox split, and 1397C represents the C-terminal G1397 DddA tox split. The boxed portion on the left side represents the DNA sequence recognized by the fusion protein (17 C or 18 C) comprising TALE and 1397C and TadA8e, and the boxed portion on the right side represents the DNA sequence recognized by the fusion protein (56 N or 57 N) comprising TALE and 1397N. The boxed "A" represents the G3460A mutation, and the intensity indicates the base-editing efficiency. The bar graph on the right shows the 3460A→G base-editing efficiency.
FIG. 6 shows the result of A-to-G base editing of the G3460A mutation in the ND1 gene of the mitochondria of a LHON patient using TALED in UDC cells. 1397N represents the N-terminal G1397 DddA tox split, and 1397C represents the C-terminal G1397 DddA tox split. The boxed portion on the left side represents the DNA sequence recognized by the fusion protein (17 C or 18 C) comprising TALE and 1397C and TadA8e, and the boxed portion on the right side represents the DNA sequence recognized by the fusion protein (56 N or 57 N) comprising TALE and 1397N. The boxed "A" represents the G3460A mutation, and the intensity indicates the base-editing efficiency. The bar graph on the right shows the 3460A→G base-editing efficiency.

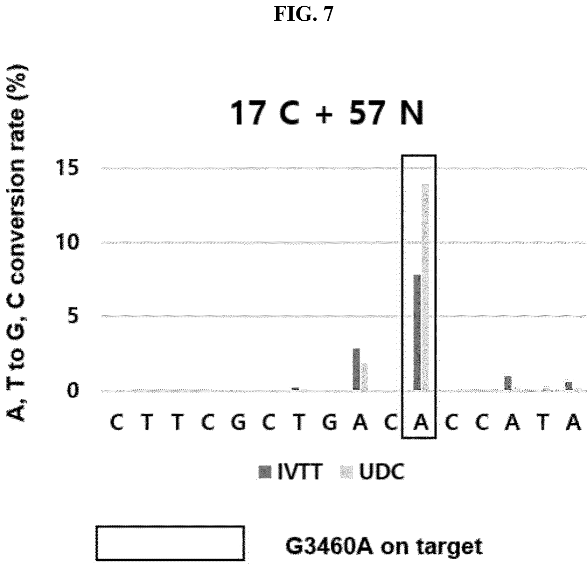

FIG. 7 shows a comparison in a bar graph between the cell-free base-editing efficiency obtained using the fusion proteins 17 C and 57 N shown in FIG. 5 used as base editors, and the intracellular base-editing efficiency obtained using the same fusion proteins shown in FIG. 6.

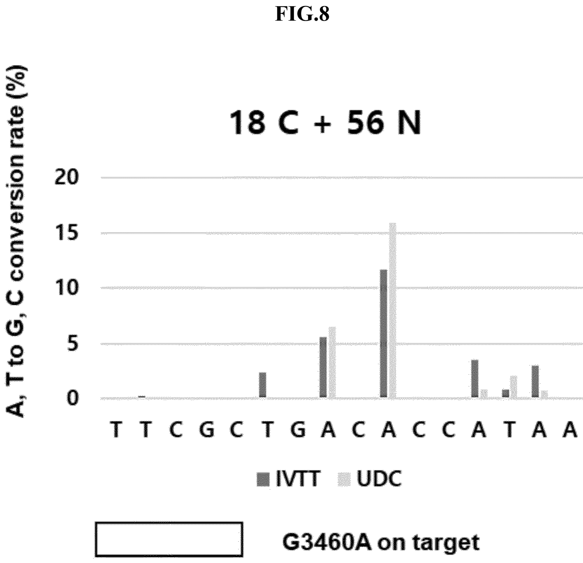

FIG. 8 shows a comparison in a bar graph between the cell-free base-editing efficiency obtained using the fusion proteins 18 C and 56 N shown in FIG. 5 used as base editors, and the intracellular base-editing efficiency obtained using the same fusion proteins shown in FIG. 6.

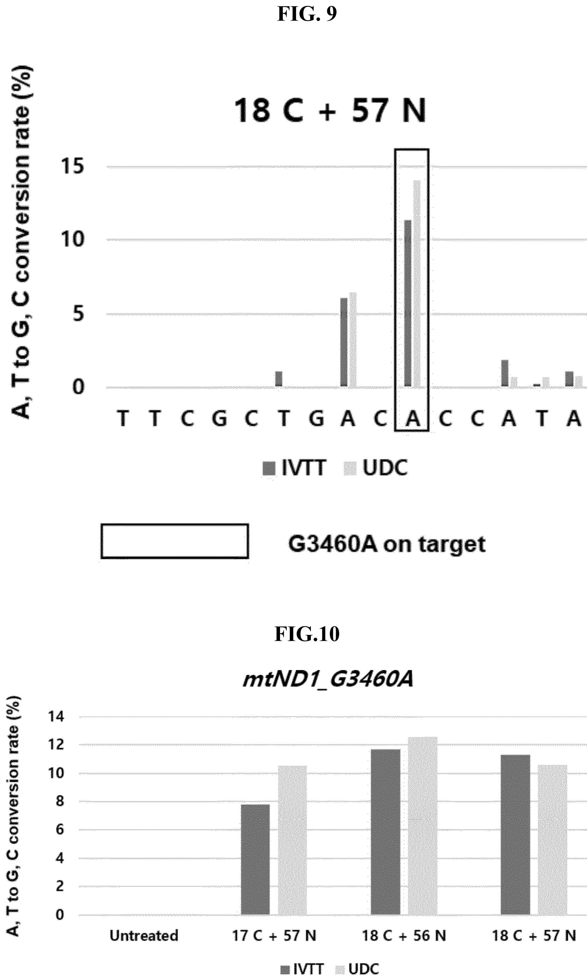

FIG. 9 shows a comparison in a bar graph between the cell-free base-editing efficiency obtained using the fusion proteins 18 C and 57 N shown in FIG. 5 used as base editors, and the intracellular base-editing efficiency obtained using the same fusion proteins shown in FIG. 6.
FIG. 10 shows a comparison in a bar graph between the 3460A→G base-editing efficiency obtained using the base editors shown in FIG. 5 and the 3460A→G base-editing efficiency obtained using the same base editors shown in FIG. 6.

### [Best Mode for Carrying out the Invention]

Unless defined otherwise, all technical and scientific terms used herein have the same meaning as commonly understood by one of ordinary skill in the art to which this invention belongs. In general, the terms used herein are well known and commonly used in the art.

The terms "correct," "edit," and "editing" as used herein are used interchangeably and refer to a method of altering a nucleic acid sequence by selective mutation of a specific genomic target. Such a specific genomic target includes, but are not limited to, a gene, a promoter, an open reading frame or any nucleic acid sequence.

The terms "base editor," "base editing system," and "base correction system" as used herein are used interchangeably, and means a substance capable of altering a nucleic acid sequence by selective mutation of a genomic target, and comprises a combination of one or more different base editors. The term "base editor", "base editing system" or "base correction system" as used herein may be in the form of a polypeptide (which may be a fusion protein) or a polynucleotide, or a combination thereof, depending on the context, and may be a composition comprising one or more polypeptides (which may be fusion proteins) or polynucleotides, or a combination thereof. Therefore, the term "base-editing system" or "base-editing composition" as used herein may comprise one base editor or a combination of two or more different base editors, which may be used simultaneously or separately.

The ability of the base-editing system to correct a target DNA mutation according to the present invention is provided by a base-editing protein, which is also called a "fusion protein" because it comprises at least a DNA binding protein and a base-converting protein. The "base-editing system" or "base editor" used in the cell-free test method according to the present invention may be in the form of one or more fusion proteins, may be in the form of a polynucleotide (DNA or mRNA) encoding the one or more fusion proteins, or may be a combination of a protein and a polynucleotide (e.g., comprising a base-editing protein (fusion protein) and a guide RNA).

The term "target" or "target site" as used herein means a pre-identified nucleic acid sequence of any composition and/or length. Such a target site includes, but are not limited to, a gene, a promoter, an open reading frame or any nucleic acid sequence.

As used herein, the terms "cell-free, in vitro,""cell-free in vitro system," or simply "cell-free" refer to a test environment that does not involve the use of cells (including tissues, organs, or organisms comprising cells), and the term "cell-free test method" means a test method that does not involve the use of cells (including tissues, organs, or organisms comprising cells). The term "in vitro coupled transcription/translation", "IVTT" or "in vitro transcription and translation reaction" as used herein means a system in which a polynucleotide (DNA or mRNA) encoding a base-editing protein is transcribed or transcribed and translated under a "cell free, in vitro system", thereby exhibiting base-editing activity as a functional base-editing protein.

The present invention provides
a method for providing information on the ability of a base-editing system to correct a DNA mutation, the method comprising:
(a) contacting an isolated DNA comprising a target DNA mutation with one or more base-editing proteins used in a base-editing system in a cell-free environment;
(b) sequencing the DNA obtained from step (a) to measure the base-editing activity of the base-editing system; and
(c) providing information on the ability of the base-editing system to correct the same DNA mutation in a cell.

The information provided by the above method may be the ability of the base editing system to correct a DNA mutation. As used herein, the term "ability to correct a DNA mutation" means the ability to correct a DNA mutation in actual cells. The information regarding the "ability to correct a DNA mutation" may be qualitative information regarding whether or not the base editing system induces a DNA mutation of interest (or an off-target mutation), or may be quantitative information regarding the extent to which the base editing system induces such a DNA mutation. The quantitative information may indicate, for example, that a DNA base is edited with an efficiency of 0.5% or more, 1% or more, 2% or more, 3% or more, 4% or more, 5% or more, 6% or more, 7% or more, 8% or more, 9% or more, 10% or more, 11% or more, 12% or more, 13% or more, 14% or more, 15% or more, 16% or more, 17% or more, 18% or more, 19% or more, or 20% or more.

The base-editing efficiency may be expressed as the percentage of sequencing reads that reflect the result of base-editing among the total sequencing reads for the DNA on which base editing was performed, and may also be expressed as the base-editing frequency.

The method of providing information on the ability of a base editing system to correct a DNA mutation according to the present invention may be part of a method of screening base editors that may be used in cells.

Specifically, the present invention may provide a method for screening a base editor capable of correcting a target DNA mutation in cells, comprising the following steps.
(a) contacting an isolated DNA comprising a target DNA mutation with candidate base editors in a cell-free environment, wherein the candidate base editors each independently comprise one or more base-editing proteins or a polynucleotide encoding the same;
(b) measuring the base editing activity of the candidate base editors by sequencing the DNA molecules obtained from step (a);
(c) providing information on the ability of the candidate base editors to correct the same DNA mutation in cells; and
(d) selecting a base editor capable of correcting the same DNA mutation in cells based on the information obtained from step (c).

The term "measurement" as used herein encompasses quantitatively determining the frequency with which a base mutation occurs, as well as qualitatively determining whether a base mutation has occurred. Whether or not a base mutation has occurred may typically be determined using DNA sequencing techniques, which are widely known in the field of biotechnology.

When the term "contacting" herein means contacting an isolated DNA comprising a target DNA mutation with one or more base-editing proteins used in a base-editing system in a cell-free environment, it includes not only adding the isolated DNA comprising the target DNA mutation and the base-editing protein as reactants to a reaction tube, but also mixing a polynucleotide encoding the base-editing protein in the form of DNA or mRNA (or a vector including the same) with the isolated DNA comprising the target DNA mutation, so that the base-editing protein obtained as a product of an in vitro transcription and/or translation process reacts with the target DNA.

In a method for providing information on the ability of a base-editing system to correct a DNA mutation according to the present invention, the step of contacting an isolated DNA comprising a target DNA mutation with a base-editing protein may involve mixing the isolated DNA comprising the target DNA mutation with one or more polynucleotides encoding one or more base-editing proteins, and allowing the polynucleotides (including DNA or mRNA) to be transcribed and translated in a cell-free environment.

The base-editing protein used in the present invention may be mixed in one reaction tube with the target DNA in the form of a polynucleotide (including DNA or mRNA) or a vector comprising the same, and used in a reaction for base-editing protein expression and DNA base editing without a separate protein purification process. For example, an expression plasmid may be used, and the plasmid may include, but is not limited to, a T7 promoter or an SP6 promoter. For example, the above method may be performed by mixing plasmid DNA and target DNA together with reactants for in vitro transcription and translation in a single reaction tube without separately purifying the synthesized protein. Expression of base-editing proteins and DNA base editing may be accomplished via a one-pot reaction in a single tube.

A reaction system for a cell-free test method according to the present invention may include factors and enzymes for in vitro transcription and translation reactions, other enzymes required for the reaction system, various substrates, buffers and salts, and may additionally include enzymes and reactants related to DNA replication required for a DNA editing process. Components included in the above reaction system may include, for example, one or more selected from: DNA polymerase; RNA polymerase; NTPs (ribonucleotide triphosphates) for the four bases (adenine, cytosine, guanine, and uracil); a cap analogue; a ribonuclease inhibitor; a protease inhibitor; an amino acid mixture; tRNA; aminoacyl-tRNA synthetase; methionyltRNA transformylase; a ribosome comprising rRNA; an initiation factor; an elongation factor; a termination factor; pyrophosphatase; MgCl₂; an antioxidant; a buffer; and a polyamine. All or part of these components (particularly those required for protein synthesis) may be provided in the form of a cell lysate (e.g., a reticulocyte lysate).

Transcription and translation reactions that may be used in the cell-free test method according to the present invention may be performed under conventional reaction conditions. For example, the reaction may be carried out at 20 to 40°C for 4 to 20 hours, and preferably, the reaction may be carried out at 30°C for 6 hours, followed by a reaction at 37°C for 16 hours.

Examples of enzymes that may be added in addition to factors and enzymes for transcription or translation reactions include, but are not limited to, enzymes for energy regeneration in the reaction system, such as creatine kinase, myokinase, and nucleoside diphosphate kinase, and enzymes for hydrolyzing inorganic pyrophosphate which are formed during transcription or translation reactions, such as inorganic pyrophosphatase.

Examples of various substrates include, but are not limited to, standard amino acids, energy sources such as nucleotide triphosphate, creatine phosphate, and formylfolate. Nucleotide triphosphates include ATP, GTP, CTP, and UTP.

Examples of buffers that may be used include, but are not limited to, a potassium phosphate buffer. Examples of salts that may be used include, but are not limited to, potassium glutamate, ammonium chloride, magnesium acetate, calcium chloride, putrescine, spermidine, and dithiothreitol.

In the cell-free test method according to the present invention, the step of contacting an isolated DNA comprising a target DNA mutation with a base-editing protein includes correcting the target DNA mutation by the base-editing protein. The base-editing reaction is performed by the DNA replication process after contact between the base-editing protein and the target DNA.

When the base-editing protein used in the cell-free test method according to the present invention is provided in the form of a polynucleotide (including DNA or mRNA) or a vector comprising the same, the expression of the base-editing protein and DNA base editing may be performed through a one-pot reaction in a single tube. For example, after completing the reaction for the in vitro transcription and translation reaction, the reaction tube may be directly used for PCR.

In a method for providing information on the ability of a base-editing system to correct a DNA mutation according to the present invention, the step of contacting an isolated DNA comprising a target DNA mutation with a base-editing protein may involve mixing the isolated DNA with the base-editing protein in a cell-free environment. In the cell-free test method according to the present invention, the step of contacting an isolated DNA comprising a target DNA mutation with a base-editing protein includes correcting the target DNA mutation by the base-editing protein. The base-editing reaction is performed by the DNA replication process after contact between the base-editing protein and the target DNA.

In this case, the base-editing protein used in the present invention may be expressed separately and/or purified, and then mixed with the target DNA. Methods for expressing and purifying a base-editing protein are well known to those skilled in the art of biotechnology.

One aspect of the present invention relates to an IVTT kit (composition) for use in a cell-free base-editing ability test method according to the present invention. The kit may include factors and enzymes for in vitro transcription and translation reactions, other enzymes required for the reaction system, various substrates, buffers, and salts, and may additionally include enzymes and reactants related to with DNA replication required for the DNA base-editing process. Components included in the kit may include, for example, one or more selected from: DNA polymerase; RNA polymerase; NTPs (ribonucleotide triphosphates) for the four bases (adenine, cytosine, guanine, and uracil); a cap analogue; a ribonuclease inhibitor; a protease inhibitor; an amino acid mixture; tRNA; aminoacyl-tRNA synthetase; methionyltRNA transformylase; a ribosome comprising rRNA; an initiation factor; an elongation factor; a termination factor; pyrophosphatase; MgCl₂; an antioxidant; a buffer; and a polyamine. All or part of these components (particularly those required for protein synthesis) may be provided in the form of a cell lysate (e.g., a reticulocyte lysate). The cell-free test method according to the present invention may involve mixing DNA encoding one or more base-editing proteins used as a base-editing system with the kit composition, followed by reaction under conditions that allow DNA transcription, translation, and base editing.

In the present invention, the reaction products including base-edited DNA obtained through an editing reaction by a base-correcting protein (the products obtained through the step (a)) may be used directly in the step of measuring base-correcting activity through sequencing (the step (b)) without separate purification. As a DNA sequencing method, conventional techniques widely known in the field of biotechnology, such as targeted deep sequencing or Sanger sequencing, may be used.

Surprisingly, it was experimentally confirmed that the results of performing base-editing in a cell-free environment without using cells and measuring the base-editing results according to the present invention were virtually identical to the results of performing base-editing within cells and then measuring the base-editing results. Here, the fact that the base-editing results are virtually identical means that the base-editing tendency and/or frequency according to the base sequence is the same. Specifically, it was confirmed that the base sequences which were found to be edited when base editing was performed in a cell-free environment were also edited in the same manner when base editing was performed in actual cells. In addition, it was confirmed that base sequences that were base-edited at a relatively high frequency in a cell-free environment were also base-edited at a relatively high frequency when base-editing was performed in actual cells. Therefore, if it is confirmed that the desired base substitution is made at the desired sequence site when base-editing is performed for a specific target DNA in a cell-free environment according to the present invention, this means that there is a very high possibility that the desired base substitution will be made at the same sequence site in an actual cell, and therefore, it may be very effectively used as a primary testing method for various base editors. Since it has been thought that the base editing activity/ability of a base editor cannot be determined without testing using cells, a method for determining the base editing ability of a base editor through a cell-free test method such as that of the present invention has not been tested or described.

To observe the efficiency of gene editing within cells, the cells must first be cultured. In this step, depending on the type of cell and the number of combinations of substances that cause genetic editing, i.e. the number of screenings, it generally takes 1 to 3 days to secure the appropriate number of cells for transfection, and 5 to 6 days if subculture is required. On the other hand, to observe the gene editing efficiency under a cell-free condition using an IVTT kit, simply mix the target DNA and the DNA encoding the gene editing protein into the IVTT kit composition, incubate at 30°C for 6 hours or 37°C for 16 hours, for example, and perform PCR. The incubation may also be performed in a PCR instrument. While cell experiments require about 3 to 12 days, cell-free experiments using the IVTT system of the present invention may be performed in just one day.

In the subsequent library production process, if the base editing ability is measured using cells, a process of lysing the cells is required, and a nested first PCR is performed using the cells as a template, a second PCR is performed using the first PCR product as a template, and a third PCR is performed using the second PCR product as a template with an indexcontaining primer to add the final index sequence. However, under a cell-free condition using the IVTT system, it is sufficient to incubate at 30 °C for 6 hours and at 37 °C for 16 hours using the above PCR system, and then proceed with the second PCR using the obtained product as a template.

In this way, the IVTT system of the present invention has the advantage of being simpler and enabling a shorter experimental time compared to experiments using cells. Even when the purpose is to develop therapeutics, mutated cells are generally required, and the methods and conditions for transfecting genetic correction agents into the cells often vary depending on the cell. On the other hand, the IVTT system of the present invention has the advantage of being able to directly produce mutated DNA and eliminates the need for condition optimization because the transfection process is omitted.

The target DNA used in the present invention may contain one or more single nucleotide genetic mutations. Verifying the base editing ability of a base-editing system to correct an inherited single nucleotide mutation is particularly difficult because it requires isolating cells from a plant or animal that harbors a relevant genetic mutation (e.g., a patient with a genetic disease). Therefore, the fact that substantially the same base-editing activity can be confirmed by performing only the cell-free test method according to the present invention, as observed when base editing is performed within a cell, may be particularly useful in developing technologies for correcting genetic mutations, such as those found in genetic diseases.

The isolated target DNA used in the present invention may be nuclear DNA or organelle DNA. The organelle DNA may be mitochondrial DNA or chloroplast DNA, and may be extracted and amplified from cells of an animal or plant, or may be synthesized. For example, the isolated target DNA may be nuclear DNA or mitochondrial DNA of a human having a genetic disease. Methods for extracting and amplifying double-stranded DNA from cells or chemically synthesizing the double-stranded DNA based on its sequence are widely known to those skilled in the art of biotechnology.

A base-editing system capable of measuring the base editing ability through a cell-free test method according to the present invention may have an activity for editing an adenine (A) base to guanine (G), cytosine (C), or thymine (T); an activity for editing a guanine (G) base to adenine (A), cytosine (C), or thymine (T); an activity for editing a cytosine (C) base to adenine (A), guanine (G), or thymine (T); an activity for editing a thymine (T) base to adenine (A), guanine (G), or cytosine (C); or may have one or more of these activities. For example, the base-editing system capable of measuring the base editing ability through the cell-free test method according to the present invention may have an activity for editing an adenine (A) base to guanine (G); an activity for editing a cytosine (C) base to thymine (T); or may have both activities.

One or more base-editing proteins included in the base-editing system that may be used in the cell-free test method according to the present invention may each independently include one or more base-converting enzymes. The base-editing protein included in the base-editing system that may be used in the cell-free test method according to the present invention is characterized in that it does not cause double-strand breaks in target DNA.

The term "base-converting enzyme" as used herein refer to an enzyme that catalyzes a reaction that converts a substituent on the purine or pyrimidine ring of a DNA base into another group or atom, or catalyzes a reaction that hydrolyzes an N-glycosidic bond in DNA to remove a DNA base, so that the target base is eventually converted (edited) into a different type of base (via the DNA replication and/or repair machinery). A type of base-converting enzyme that catalyzes a reaction in which a substituent on the purine or pyrimidine ring of a DNA base is converted into another group or atom includes a deaminase, and a type of base-converting enzyme that catalyzes a reaction in which an N-glycosidic bond of DNA is hydrolyzed to remove a DNA base includes a DNA glycosylase.

For example, one or more base-editing proteins that may be included in the base-editing system used in the cell-free test method according to the present invention may each independently include cytosine deaminase and/or adenine deaminase as base-converting enzymes.

The cytosine deaminase that may be used in the base-editing system used in the cell-free test method according to the present invention means any deaminase enzyme having an activity of converting a cytosine base into uridine, and may be derived from or mutated (e.g., engineered or evolved) from any organism (e.g., eukaryote or prokaryote), including, but not limited to, algae, bacteria, fungi, plants, invertebrates, and mammals. For example, the cytosine deaminase may be a cytosine deaminase derived from or mutated from APOBEC (apolipoprotein B editing complex), AID (activation-induced deaminase), TadA (tRNA-specific adenosine deaminase), a bacterial adenine deaminase, or an ortholog thereof, or a cytosine deaminase derived from or mutated from DddA, a bacterial cytosine deaminase, or an ortholog thereof, or a split thereof. The cytosine deaminase mutated from the above-mentioned TadA may be, for example, one in which one or more of the amino acid residues 6, 26, 27, 28, 46, 48, 49, 61, 74, 76, 77, 82, 96, 107, 108, 112, 114, 115, 119, 122, 127, 142, 143, 151, 154 and 158 of the amino acid sequence of SEQ ID NO: 1 are substituted with other amino acids. For example, it may be a polypeptide in which the amino acid at position 27 of SEQ ID NO: 1 is substituted with lysine, the amino acid at position 28 is substituted with alanine, the amino acid at position 61 is substituted with isoleucine, and the amino acid at position 96 is substituted with asparagine. Regarding the composition of the cytosine deaminase that may be used in the present invention, reference may be made to international patent application publications WO 2022/060185, WO 2023/086953, etc., which are herein incorporated by reference in their entirety, and which describe technologies known prior to the filing of the present application.

When a cytosine deaminase is included in the base-editing system used in the cell-free test method according to the present invention, the cytosine deaminase may be present in the form of first and second splits, or in a full-length form. In the case where the cytosine deaminase may be present in the form of first and second splits, the first split and the second split are linked to different DNA binding proteins.

In this specification, when two proteins are described as being "linked," they may be directly linked, or indirectly linked via a linker or other protein(s).

The cytosine deaminase used in the present invention may be DddA tox, which is a portion of a bacterial toxin derived from Burkholderia cenocepacia that exhibits an enzymatic function and may deaminate cytosine in double-stranded DNA. DddA tox may comprise the amino acid sequence of SEQ ID NO: 2.

SEQ ID NO: 2: wild-type DddA tox

GSYALGPYQISAPQLPAYNGQTVGTFYYVNDAGGLESKVFSSGGPTPYPNYA

NAGHVEGQSALFMRDNGISEGLVFHNNPEGTCGFCVNMTETLLPENAKMTVVPPEG
AIPVKRGATGETKVFTGNNSNSPKSPTKGGC

Since DddA tox is toxic to cells, it may be employed in the form of two inactive splits, namely a first split and a second split, in order to avoid toxicity in a host cell. When the cytosine deaminase used in the present invention is employed in the form of first and second splits, each of the splits lacks deamination activity.

The first split of the DddA tox cytosine deaminase may include a sequence from the N-terminus to one of G33, G44, A54, N68, G82, N98, or G108 in the amino acid sequence of SEQ ID NO: 2, and the second split may include a sequence from one of G34, P45, G55, N69, T83, A99, or A109 to the C-terminus in the amino acid sequence of SEQ ID NO: 2.

Preferably, the first split of the DddA tox cytosine deaminase may comprise a sequence from the N-terminus up to G44 of the amino acid sequence of SEQ ID NO: 2 (SEQ ID NO: 3 as set forth below), and the second split may comprise a sequence from P45 to the C-terminus (SEQ ID NO: 4 as set forth below).
SEQ ID NO: 3: wild-type DddA toxG1333-N
   GSYALGPYQISAPQLPAYNGQTVGTFYYVNDAGGLESKVFSSGG
SEQ ID NO: 4: wild-type DddA toxG1333-C

PTPYPNYANAGHVEGQSALFMRDNGISEGLVFHNNPEGTCGFCVNMTETLLPENAK
MTVVPPEGAIPVKRGATGETKVFTGNSNSPKSPTKGGC

Preferably, the first split of the DddA tox cytosine deaminase may comprise the sequence from the N-terminus to G108 of the amino acid sequence of SEQ ID NO: 2 (SEQ ID NO: 5 as set forth below), and the second split may comprise the sequence from A109 to the C-terminus (SEQ ID NO: 6 as set forth below).
SEQ ID NO: 5: wild-type DddA toxG1397-N 
GSYALGPYQISAPQLPAYNGQTVGTFYYVNDAGGLESKVFSSGGPTPYPNYA
NAGHVEGQSALFMRDNGISEGLVFHNNPEGTCGFCVNMTETLLPENAKMTVVPPEG
SEQ ID NO: 6: wild-type DddA toxG1397-C
   AIPVKRGATGETKVFTGNNSNSPKSPTKGGC

When the first and second splits of DddA tox are used as cytosine deaminase, one or more amino acids located on the surface where the first and second splits of the cytosine deaminase bind to each other may be substituted with another amino acid. For example, the first split and the second split of DddA tox may each comprise the amino acid sequences of SEQ ID NO: 3 (G1333-N) and SEQ ID NO: 4 (G1333-C), respectively, in which case at least one amino acid selected from the group consisting of positions 3, 5, 10, 11, 13, 14, 15, 16, 17, 18, 19, 28, 30, and 31 of SEQ ID NO: 3, or at least one amino acid selected from the group consisting of positions 13, 16, 17, 20, 21, 28, 29, 30, 31, 32, 33, 56, 57, 58, and 60 of SEQ ID NO: 4, may be substituted with another amino acid, but is not limited thereto. As another example, the first split of DddA tox may comprise the amino acid sequence of SEQ ID NO: 5 (G1397-N), and the second split may comprise the amino acid sequence of SEQ ID NO: 6 (G1397-C), wherein at least one amino acid selected from the group consisting of positions 87, 88, 91, 92, 95, 100, 101, 102, and 103 of SEQ ID NO: 5 or at least one amino acid selected from the group consisting of positions 13, 14, 15, and 16 of SEQ ID NO: 6 may be substituted with another amino acid, but is not limited thereto. The above "another amino acid" means an amino acid selected from among alanine, isoleucine, leucine, methionine, phenylalanine, proline, tryptophan, valine, cysteine, glutamine, glycine, serine, threonine, tyrosine, glutamic acid, arginine, histidine, lysine, and the known variants of the above amino acids, excluding the amino acid originally present at the mutant position in the wild-type protein. Using such a mutant, the pair of two DddA tox splits, each linked to a DNA-binding protein, fails to function properly if they fail to bind to DNA, thereby enabling highly efficient and precise C-to-T editing without causing undesired off-target C-to-T editing. Examples of such a mutant include a first split of DddA tox having the amino acid sequence of SEQ ID NO: 7 (which may be referred to as "G1397-N" or "G1397N") and a second split of DddA tox having the amino acid sequence of SEQ ID NO: 8 (which may be referred to as "G1397-C" or "G1397C").

In the present specification, the term "G1333-N", "G1333N", or "1333N" may refer to a first split of wild-type DddA tox having the amino acid sequence of SEQ ID NO: 3 or an amino acid variant thereof, and the term "G1333-C", "G1333C", or "1333C" may refer to a second split of wild-type DddA tox having the amino acid sequence of SEQ ID NO: 4 or an amino acid variant thereof.

In the present specification, the term "G1397-N", "G1397N" or "1397N" may mean a first split of wild-type DddA tox having an amino acid sequence of SEQ ID NO: 5 or 7 or an amino acid variant thereof, and the term "G1397-C", "G1397C" or "1397C" may mean a second split of wild-type DddA tox having an amino acid sequence of SEQ ID NO: 6 or 8 or an amino acid variant thereof.

The cytosine deaminase used in the base-editing system employed in the cell-free test method according to the present invention may be used in a full-length form, and the full-length cytosine deaminase used in this case (e.g., DddA tox) has an amino acid sequence modified to reduce or eliminate toxicity. The C-terminus of DddA tox is specifically enriched with positively charged amino acids. Because DNA is negatively charged, it binds to positively charged amino acids in proteins. By substituting these positively charged amino acids, the binding strength of DddA tox to DNA may be weakened, thereby reducing or eliminating intracellular toxicity. That is, if a positively charged amino acid is substituted to eliminate toxicity, cloning using E. coli becomes possible, thereby allowing the preparation of full-length DddA tox. Such non-toxic full-length cytosine deaminase may be provided by substituting one or more, two or more, three or more, four or more, or five or more amino acids of the wild-type amino acid sequence of SEQ ID NO: 2 with other amino acids. The "other amino acids" mean amino acids selected from among alanine, isoleucine, leucine, methionine, phenylalanine, proline, tryptophan, valine, aspartic acid, cysteine, glutamine, glycine, serine, threonine, tyrosine, glutamic acid, arginine, histidine, lysine, and known variants thereof, excluding the amino acid originally present at the mutant position in the wild-type protein. For example, the other amino acids may comprise alanine.

For example, a full-length cytosine deaminase variant that may be included in the base-editing system used in the cell-free test method according to the present invention may have one or more amino acid substitutions selected from the group consisting of a substitution of S at position 37 to G, a substitution of G at position 59 to S, a substitution of A at position 109 to V, and a substitution of S at position 129 to G in the amino acid sequence of SEQ ID NO: 2.

For example, a full-length cytosine deaminase variant that may be included in the base-editing system used in the cell-free test method according to the present invention may have all of the following substitutions: S to G at position 37, G to S at position 59, A to V at position 109, and S to G at position 129 in the amino acid sequence of SEQ ID NO: 2.

The adenine deaminase that may be used in the base-editing system used in the cell-free test method according to the present invention refers to an amino group deaminase having activity of converting an adenine base into hypoxanthine (or inosine as a nucleoside), and may be derived from or mutated (e.g., engineered or evolved) from any organism including, but not limited to, algae, bacteria, fungi, plants, invertebrates, and mammals, such as E. coli, S. aureus, S. typhi, S. putrefaciens, H. influenzae, or C. crescentus. Such adenine deaminase may be, for example, APOBEC, AID, or TadA, or a variant thereof. The aforementioned TadA may be, for example, TadA8e (SEQ ID NO: 1) or a truncated form or a variant thereof (for example, a variant improved or evolved to be applicable to deoxynucleotides). The above-mentioned variant of TadA8e may be, for example, one in which one or more of amino acid residues 23, 28, 30, 36, 46, 48, 49, 51, 76, 82, 84, 106, 108, 110, 111, 146, 147, 152, 154, 155, 156 and 157 of SEQ ID NO: 1 are substituted with other amino acids. Regarding the composition of adenine deaminase that may be used in the present invention, reference may be made to international patent application publications WO 2022/060185, WO 2023/086953, etc., which are incorporated by reference in their entirety into this application, and which were already known prior to the present application. An adenine deaminase that may be used in the base-editing system used in the cell-free test method according to the present invention may include an amino acid sequence of SEQ ID NO: 1 or a conservative amino acid substitution thereof.

One or more base-editing proteins included in the base-editing system used in the cell-free test method according to the present invention may each independently include cytosine deaminase (or a split thereof) or adenine deaminase, or both.

One or more base-editing proteins included in the base-editing system that may be used in the cell-free test method according to the present invention may each independently include one or more DNA binding proteins selected from the group consisting of a zinc finger protein, a TALE (transcriptional activator-like effector) protein, a CRISPR-associated nuclease, and a CRISPR-associated nickase. The base-editing protein included in the base-editing system that may be used in the cell-free test method according to the present invention is characterized in that it does not cause double-strand breaks in target DNA.

When the base-editing system used in the present invention is in the form of a composition comprising two or more different base editors, one base editor may comprise a zinc finger protein, a TALE protein, a CRISPR-associated nuclease, or a CRISPR-associated nickase, and another base editor may independently comprise a zinc finger protein, a TALE protein, a CRISPR-associated nuclease, or a CRISPR-associated nickase. Regarding the compositions of zinc finger proteins, TALE proteins, CRISPR-associated nucleases and CRISPR-associated nickases, reference may be made to international patent application publications WO 2022/060185 and WO 2022/017745, which are incorporated by reference herein in their entirety, and which were already known prior to the present application.

Zinc fingers are a representative DNA-binding protein structure that forms a major DNA-binding protein motif, and the interaction between the α-helix of the zinc finger and the major groove of DNA enables strong and specific recognition of DNA sequences. One or more zinc finger motifs may be used in combination.

The DNA binding protein used in the base editing system that may be used in the cell-free test method according to the present invention may be a TALE protein. The TALE protein of the present invention means a protein that binds to nucleotides in a sequence-specific manner via one or more TALE-repeat modules. The TALE protein comprises at least one TALE-repeat module, preferably 1 to 30 TALE-repeat modules, but is not limited thereto. The TALE-repeat modules may be referred to as a "TALE array", and the term "TALE protein" means a configuration comprising an N-terminal domain and a C-terminal domain (which may include a half domain) on each side of the TALE array. As used herein, the term "TALE" means "TALE protein" unless otherwise indicated.

When a TALE protein is included as a DNA-binding protein in a base-editing system that may be used in a cell-free test method according to the present invention, a single-module TALE array or a multiple-module TALE array (e.g., a dual-module TALE array of a first TALE (or left TALE) array and a second TALE (or right TALE) array) may be used.

When the base-editing system that may be used in the cell-free test method according to the present invention comprises two fusion proteins, each comprising a TALE protein, the two fusion proteins may comprise a first TALE protein and a second TALE protein, respectively. The first TALE protein and the second TALE protein may each independently be linked, directly or indirectly (e.g., via a linker and/or another protein component), to at least one of one or more base-converting enzymes, e.g., cytosine deaminase and adenine deaminase. For example, a first fusion protein comprising a first TALE protein (left TALE) (a fusion protein that binds DNA 5' upstream of the base-editing target site) may include a first split of cytosine deaminase, a second fusion protein comprising a second TALE protein (right TALE) may include a second split of cytosine deaminase, and either or both of the first fusion protein and the second fusion protein may include an adenine deaminase. Alternatively, the first fusion protein comprising the first TALE protein (left TALE) may comprise a second split of cytosine deaminase, the second fusion protein comprising the second TALE protein (right TALE) may comprise a first split of cytosine deaminase, and either or both of the first fusion protein and the second fusion protein may comprise an adenine deaminase. Alternatively, the full-length form of cytosine deaminase may be included in either the first fusion protein comprising the first TALE protein or the second fusion protein comprising the second TALE protein, and either or both of the first fusion protein and the second fusion protein may include an adenine deaminase.

The DNA binding protein used in the base editing system that may be used in the cell-free test method according to the present invention may be a CRISPR-associated protein called a "Cas protein". The "CRISPR system," well known as a DNA editing tool, refers to a ribonucleoprotein complex in which the Cas protein and associated RNA component(s) work together to recognize specific bases in DNA and perform the function of editing the corresponding site, and is also called the "CRISPR/Cas system".

The term "Cas protein" refers to an essential protein component of the CRISPR/Cas system. The CRISPR-associated (cas) genes encoding Cas proteins are often associated with a CRISPR repeat-spacer array. More than 40 different Cas protein families have been described, and information on known Cas genes and proteins is available from, but not limited to, GenBank at the National Center for Biotechnology Information (NCBI). The Cas proteins may be isolated from microorganisms such as bacteria, or may be produced by non-natural processes, such as recombinant synthesis or artificial synthesis.

The DNA binding protein used in the base editing system that may be used in the cell-free test method according to the present invention may be a CRISPR-associated nuclease. Here, the term "CRISPR-associated nuclease" refers to any Cas protein or a variant thereof having (endo)nuclease activity when complexed with a guide RNA. When a CRISPR-associated nuclease is used as part of a base-editing protein in the cell-free test method according to the present invention, a guide RNA may be added when the isolated DNA, which contains a target DNA mutation, is contacted with the base-editing protein or a polynucleotide (DNA or mRNA) encoding the same.

The term "guide RNA" as used herein refers to an RNA specific for a target DNA, which may form a complex with a Cas protein and bring the Cas protein to the target DNA. The guide RNA may be composed of two RNAs, namely, CRISPR RNA (crRNA) and transactivating crRNA (tracrRNA), or may be a single-chain RNA (sgRNA) generated by the fusion of essential parts of crRNA and tracrRNA. The guide RNA may be a dual RNA comprising crRNA and tracrRNA. Any guide RNA may be used in the cell-free test method according to the present invention, provided that the guide RNA comprises essential portions of crRNA and tracrRNA and a portion complementary to the target.

The DNA binding protein used in the base editing system that may be used in the cell-free test method according to the present invention may be a CRISPR-associated nickase. Here, the term "CRISPR-associated nickase" means any Cas protein or a variant thereof having nickase activity when complexed with a guide RNA. The CRISPR-associated nickase used in the cell-free test method according to the present invention may be a mutant that has nickase activity and has lost part or all of its endonuclease activity for cleaving double-stranded DNA from the existing Cas protein. The "nickase" activity means the activity of cleaving only one of the two strands of a DNA molecule. Such a variant may be isolated from microorganisms, such as bacteria, or may be produced by recombinant or artificial synthesis. In a case where a CRISPR-associated nickase is used as part of a base-editing protein in the cell-free test method according to the present invention, when the isolated DNA comprising the target DNA mutation is contacted with the base-editing protein or a polynucleotide (DNA or mRNA) encoding the base-editing protein, a reverse transcriptase or a polynucleotide (DNA or mRNA) encoding the reverse transcriptase, together with a guide RNA (also referred to as a prime editing guide RNA), may be added.

When a cytosine deaminase is included in a fusion protein comprising a DNA binding protein, the cytosine deaminase may be linked directly or indirectly (e.g., via a linker and/or another protein component) to the N-terminus or C-terminus of the DNA binding protein. When an adenine deaminase is included in a fusion protein comprising a DNA binding protein, the adenine deaminase may be linked directly or indirectly (e.g., via a linker and/or another protein component) to the N-terminus or C-terminus of the TALE protein. When a fusion protein comprising a DNA binding protein comprises both a cytosine deaminase and an adenine deaminase, the adenine deaminase may be linked directly or indirectly (e.g., via a linker and/or another protein component) to the N-terminus or C-terminus (preferably the C-terminus) of the cytosine deaminase.

When the base-editing system that may be used in the cell-free test method according to the present invention comprises two fusion proteins, the fusion proteins may have different compositions and arrangements of protein components (e.g., a DNA binding protein and one or more base-converting enzymes). For example, the first fusion protein (the fusion protein that binds DNA 5' upstream from the base-editing target site) may comprise an adenine deaminase, whereas the second fusion protein (the fusion protein that binds DNA 3' downstream from the base-editing target site) may not comprise an adenine deaminase, or vice versa. For example, the first fusion protein (a fusion protein that binds DNA 5' upstream from the base-editing target site) may comprise a TALE protein, while the second fusion protein (a fusion protein that binds DNA 3' downstream from the base-editing target site) may comprise a zinc finger protein, or vice versa.

Regarding the arrangement of the protein components of the fusion protein, the first fusion protein (a fusion protein that binds to DNA 5' upstream from the base-editing target site) and the second fusion protein (a fusion protein that binds to DNA 3' downstream from the base-editing target site) may be arranged independently. For example, in the first fusion protein, the adenine deaminase may be positioned after the C-terminus of the cytosine deaminase, whereas in the second fusion protein, the adenine deaminase may be positioned before the N-terminus of the cytosine deaminase, or vice versa. For example, in the first fusion protein, the DNA binding protein may be positioned after the C-terminus of the cytosine deaminase, whereas in the second fusion protein, the DNA binding protein may be positioned before the N-terminus of the cytosine deaminase, or vice versa.

One or more fusion proteins included in the base-editing system that may be used in the cell-free test method according to the present invention may each independently further include a UGI (uracil glycosylase inhibitor). The UGI may increase base-editing efficiency by inhibiting the activity of UDG (uracil DNA glycosylase), an enzyme that repairs variant DNA by catalyzing the removal of uracil (U) bases from DNA. When UGI is used in a base-editing protein included in a base-editing system that may be used in a cell-free test method according to the present invention, its location may vary, and for example, it may be directly or indirectly linked (e.g., via a linker and/or another protein component) to the C-terminus of cytosine deaminase, but is not limited thereto.

One or more fusion proteins included in the base-editing system that may be used in the cell-free test method according to the present invention may each independently further include a nuclear export signal (NES). The NES sequence may be any signal sequence that confers the ability to translocate outside the nucleus, and a natural NES or an artificially synthesized NES may be used. For example, it may be derived from mirut virus of mice (MVM), but is not limited thereto. When an NES is used in a base-editing system that may be used in a cell-free test method according to the present invention, its location may vary and, for example, it may be directly or indirectly linked (e.g., via a linker and/or another protein component) to the N-terminus of a cytosine deaminase, but is not limited thereto.

One or more fusion proteins included in the base-editing system that may be used in the cell-free test method according to the present invention may each independently further include a mitochondrial targeting sequence (MTS). The MTS may be any signal sequence capable of translocating into mitochondria, and may be a natural MTS present at the N-terminus of various mitochondrial proteins, or alternatively, an artificially synthesized MTS may be used. When the MTS is used in a base-editing system that may be used in a cell-free test method according to the present invention, its location may vary and, for example, it may be linked directly or indirectly (e.g., via a linker and/or another protein component) to the N-terminus of a DNA binding protein or the N-terminus of an NES, but is not limited thereto.

The term "fusion protein" as used herein means a polypeptide formed by joining two or more different polypeptides via peptide bonds. A fusion protein included in a base-editing system that may be used in the cell-free test method according to the present invention comprises a DNA binding protein and a base-converting enzyme, and may further comprise one or more of UGI, NES, and MTS, and these protein components may be linked either directly or via a linker. In addition, a tag sequence may be added as needed. The method for designing and constructing a fusion protein (or a polynucleotide encoding the fusion protein) may be any method known in the art of biotechnology, and the polynucleotide may be inserted into a vector.

The present invention may relate to the following (1) to (44) in view of the foregoing description, but is not limited thereto.
(1) A method for providing information on the ability of a base editing system to correct a DNA mutation, the method comprising the steps of:
   (a) contacting an isolated DNA comprising a target DNA mutation with one or more base-editing proteins used in a base-editing system in a cell-free environment;
   (b) sequencing the DNA obtained from step (a) to measure the base-editing activity of the base-editing system; and
   (c) providing information on the ability of the base-editing system to correct the same DNA mutation in cells.
(2) The method according to (1), wherein step (a) involves mixing an isolated DNA comprising a target DNA mutation with one or more polynucleotides encoding one or more base-editing proteins, and wherein the one or more polynucleotides are transcribed and translated in a cell-free environment.
(3) The method according to (2), wherein the one or more polynucleotides encoding the one or more base-editing proteins are DNA or mRNA.
(4) The method according to any one of (1) to (3), wherein, in step (a), a base-editing protein is expressed and a DNA base is edited through a one-pot reaction.
(5) The method according to (1), wherein step (a) involves mixing an isolated DNA comprising a target DNA mutation with a base-editing protein in the cell-free environment.
(6) The method in any one of (1) to (5), wherein a reaction product of step (a) is used in step (b) without separate purification.
(7) The method according to any one of (1) to (6), wherein the target DNA mutation comprises one or more single nucleotide mutations.
(8) The method according to any one of (1) to (7), wherein the isolated DNA comprising the target DNA mutation is extracted and amplified from an animal or plant cell, or is artificially synthesized.
(9) The method according to any one of (1) to (8), wherein the isolated DNA comprising the target DNA mutation is nuclear DNA or organelle DNA.
(10) The method according to (9), wherein the organelle DNA is mitochondrial DNA or chloroplast DNA.
(11) The method according to any one of (1) to (10), wherein the isolated DNA comprising the target DNA mutation is nuclear DNA or mitochondrial DNA from a human having a genetic disease.
(12) The method according to any one of (1) to (11), wherein the base editing is performed to convert an adenine (A) base to guanine (G), a cytosine (C) base to thymine (T), or both.
(13) The method according to any one of (1) to (12), wherein the one or more base-editing proteins each independently comprise one or more DNA binding proteins selected from the group consisting of a zinc finger protein, a transcriptional activator-like effector (TALE) protein, a CRISPR-associated nuclease, and a CRISPR-associated nickase.
(14) The method according to (13), wherein the one or more DNA binding proteins comprise a CRISPR-associated nuclease, and in step (a), the guide RNA is further contacted with the isolated DNA comprising the target DNA mutation.
(15) The method according to (13), wherein the one or more DNA binding proteins comprise a CRISPR-associated nickase, and in step (a), a reverse transcriptase or a polynucleotide encoding the reverse transcriptase, and a guide RNA are further contacted with the isolated DNA comprising the target DNA mutation
(16) The method according to any one of (1) to (15), wherein one or more of the base proteins each independently comprise a base-converting enzyme.
(17) The method according to (16), wherein the one or more base-converting enzymes independently comprise one or more of APOBEC (apolipoprotein B editing complex), AID (activation-induced deaminase), TadA (tRNA-specific adenosine deaminase), or DddA tox, or a variant thereof.
(18) The method according to (17), wherein DddA tox or a variant thereof is used in the form of two splits.
(19) The method according to (17), wherein DddA tox or a variant thereof is used in the form of a full-length protein.
(20) The method according to any one of (1) to (19), wherein the one or more base-editing proteins each independently comprise a uracil glycosylase inhibitor (UGI).
(21) A method for screening a base editor capable of correcting a target DNA mutation in cells, comprising the following steps:
   (a) a step of contacting an isolated DNA comprising a target DNA mutation with candidate base editors in a cell-free environment, wherein the candidate base editors each independently comprise one or more base-editing proteins or one or more polynucleotides encoding the same;
   (b) measuring the base editing activity of the candidate base editors by sequencing the DNA molecules obtained from step (a);
   (c) providing information on the ability of the candidate base editors to correct the same DNA mutation in cells; and
   (d) selecting a base editor capable of correcting the same DNA mutation in cells based on the information obtained from step (c).
(22) In (21), wherein step (a) involves mixing an isolated DNA comprising a target DNA mutation with one or more polynucleotides encoding one or more base-editing proteins, and wherein the one or more polynucleotides are transcribed and translated in a cell-free environment.
(23) The method according to (22), wherein the one or more polynucleotides encoding the one or more base-editing proteins are DNA or mRNA.
(24) The method according to any one of (21) to (23), wherein, in step (a), a base-editing protein is expressed and a DNA base is edited through a one-pot reaction.
(25) The method according to (21), wherein step (a) involves mixing an isolated DNA comprising a target DNA mutation with a base-editing protein in the cell-free environment.
(26) The method according to any one of (21) to (25), wherein a reaction product of step (a) is used in step (b) without separate purification.
(27) The method according to any one of (21) to (26), wherein the target DNA mutation comprises one or more single nucleotide mutations.
(28) The method according to any one of (21) to (27), wherein the isolated DNA comprising the target DNA mutation is extracted and amplified from an animal or plant cell, or is artificially synthesized.
(29) The method according to any one of (21) to (28), wherein the isolated DNA comprising the target DNA mutation is nuclear DNA or organelle DNA.
(30) The method according to (29), wherein the organelle DNA is mitochondrial DNA or chloroplast DNA.
(31) The method according to any one of (21) to (30), wherein the isolated DNA comprising the target DNA mutation is nuclear DNA or mitochondrial DNA from a human having a genetic disease.
(32) The method according to any one of (21) to (31), wherein the base editing is performed to convert an adenine (A) base to guanine (G), a cytosine (C) base to thymine (T), or both.
(33) The method according to any one of (21) to (32), wherein the one or more base-editing proteins each independently comprise one or more DNA binding proteins selected from the group consisting of a zinc finger protein, a transcriptional activator-like effector (TALE) protein, a CRISPR-associated nuclease, and a CRISPR-associated nickase.
(34) The method according to (33), wherein the one or more DNA binding proteins comprise a CRISPR-associated nuclease, and in step (a), the guide RNA is further contacted with the isolated DNA comprising the target DNA mutation.
(35) The method according to (33), wherein the one or more DNA binding proteins comprise a CRISPR-associated nickase, and in step (a), a reverse transcriptase or a polynucleotide encoding the reverse transcriptase, and a guide RNA are further contacted with the isolated DNA comprising the target DNA mutation.
(36) The method according to any one of (21) to (35), wherein one or more of the base proteins each independently comprise a base-converting enzyme.
(37) The method according to (36), wherein the one or more base-converting enzymes independently comprise one or more of APOBEC (apolipoprotein B editing complex), AID (activation-induced deaminase), TadA (tRNA-specific adenosine deaminase), or DddA tox, or a variant thereof.
(38) The method according to (37), wherein DddA tox or a variant thereof is used in the form of two splits.
(39) The method according to (37), wherein DddA tox or a variant thereof is used in the form of a full-length protein.
(40) The method according to any one of claims (21) to (39), wherein the one or more base-editing proteins each independently comprise a uracil glycosylase inhibitor (UGI).
(41) A method according to any one of (1) to (40), wherein a composition comprising a DNA polymerase, an RNA polymerase, a mixture of ribonucleotide triphosphates, a mixture of amino acids, a ribosome comprising tRNA and rRNA, an initiation factor, an elongation factor, a termination factor, and a buffer is used in step (a).
(42) A base editor selected by the method according to any one of (21) to (41).
(43) A base editor selected by the method according to any one of claims (21) to (41), wherein the base editor comprises at least one base-editing protein used in step (a) or a polynucleotide encoding the same.
(44) A kit according to any one of (1) to (41), comprising a DNA polymerase, an RNA polymerase, a mixture of ribonucleotide triphosphates, a mixture of amino acids, a ribosome comprising tRNA and rRNA, an initiation factor, an elongation factor, a termination factor, and a buffer.

Hereinafter, the present invention will be described in more detail with reference to the following examples. However, the following examples are merely illustrative of the present invention and are not to be construed as limiting.

### Example 1: Amplification of template DNA

A double-stranded DNA sequence mimicking the human mitochondrial ND1 gene was synthesized as a gBlock DNA fragment from IDT (Integrated DNA Technologies) to serve as a target DNA for DNA editing. The sequence of the obtained template DNA is as follows.
TACTATTGCCAGCATTGCTGCCCTCAACCTAGGCCTCCTATTTATTCTAGCC ACCTCTAGCCTAGCCGTTTACTCAATCCTCTGATCAGGGTGAGCATCAAACTCAAA CTACGCCCTGATCGGCGCACTGCGAGCAGTAGCCCAAACAATCTCAAGATGGTGC ACGATGCACAG (The underlined sequence is an arbitrary PCR amplification sequence used to prevent contamination.)

The template DNA was amplified using a forward primer (TACTATTGCCAGCATTGCTGC) and a reverse primer (CTGTGCATCGTGCACCATCT), and then purified using a PCR purification kit (Geneall). Immediately before the experiment, the DNA was freshly diluted in distilled water to a concentration of 10 ng/µl and used.

### Example 2: In vitro transcription and translation (IVTT) and base editing

After quantifying the template DNA obtained in Example 1 to a concentration of 10 ng/µL, a reaction mixture (with distilled water added up to 25 µL), which contained the template DNA (1 ng, 5 ng, or 10 ng), 0.5 µg of a plasmid comprising DNA encoding the left DdCBE (or sTALED), 0.5 µg of a plasmid comprising DNA encoding the right DdCBE (or sTALED), and 20 µL of an in vitro coupled transcription/translation (IVTT) kit mixture, was mixed in a tube, incubated at 30°C for 6 hours, and then incubated at 37°C for 16 hours. The IVTT kit used was the TNT^{®} Quick Coupled Transcription/Translation System (Promega), and the TNT^{®} Quick Master Mix, which includes TNT^{®} Rabbit Reticulocyte Lysate, TNT^{®} Reaction Buffer, TNT^{®} RNA Polymerase, Amino Acid Mixture, and RNasin^{®} Ribonuclease Inhibitor, was used.

The amino acid sequences of the left DdCBE. left sTALED, right DdCBE, and right sTALED are as follows. Each of the proteins is located between the T7 promoter (TAATACGACTCACTATAG) and the terminator sequence.
Left DdCBE: 
MALSRAVCGTSRQLAPVLGYLGSRQKHSLPDYPYDVPDYAGYPYDVPDYAG
YPYDVPDYAGIRIQDLRTLGYSQQQQEKIKPKVRSTVAQHHEALVGHGFTHAHIVALS
QHPAALGTVAVKYQDMIAALPEATHEAIVGVGKQWSGARALEALLTVAGELRGPPLQ
LDTGQLLKIAKRGGVTAVEAVHAWRNALTGAPLNLTPDQVVAIASHDGGKQALETVQ
RLLPVLCQAHGLTPDQVVAIASNGGGKQALETVQRLLPVLCQDHGLTPAQVVAIASNI
GGKQALETVQRLLPVLCQDHGLTPDQVVAIASNNGGKQALETVQRLLPVLCQAHGL
TPDQVVAIASHDGGKQALETVQRLLPVLCQDHGLTPDQVVAIASHDGGKQALETVQR
LLPVLCQAHGLTPAQVVAIASNGGGKQALETVQRLLPVLCQDHGLTPAQVVAIASNIG
GKQALETVQRLLPVLCQAHGLTPDQVVAIASNNGGKQALETVQRLLPVLCQAHGLTP
AQVVAIASHDGGKQALETVQRLLPVLCQDHGLTPDQVVAIASHDGGKQALETVQRLL
PVLCQAHGLTPEQVVAIASNNGGKQALETVQRLLPVLCQAHGLTPAQVVAIASNGGG
KQALETVQRLLPVLCQAHGLTPAQVVAIASNGGGKQALETVQRLLPVLCQAHGLTPE
QVVAIASNGGGKQALESIVAQLSRPDPALAALLVGSGSYALGPYQISAPQLPAYNGQT
VGTFYYVNDAGGLESKVFSSGGPTPYPNYANAGHVEGQSALFMRDNGISEGLVFHN
NPEGTCGFCVNMTETLLPENAKMTVVPPEGSGGSTNLSDIIEKETGKQLVIQESILMLP
EEVEEVIGNKPESDILVHTAYDESTDENVMLLTSDAPEYKPWALVIQDSNGENKIKML
(SEQ ID NO: 9)
Right DdCBE: 
MASVLTPLLLRGLTGSARRLPVPRAKIHSLDYKDHDGDYKDHDIDYKDDDD
KGIRIQDLRTLGYSQQQQEKIKPKVRSTVAQHHEALVGHGFTHAHIVALSQHPAALGT
VAVKYQDMIAALPEATHEAIVGVGKQWSGARALEALLTVAGELRGPPLQLDTGQLLK
IAKRGGVTAVEAVHAWRNALTGAPLNLTPDQVVAIASNNGGKQALETVQRLLPVLCQ
 
DHGLTPAQVVAIASNIGGKQALETVQRLLPVLCQAHGLTPEQVVAIASNNGGKQALET
VQRLLPVLCQAHGLTPDQVVAIASNGGGKQALETVQRLLPVLCQAHGLTPAQVVAIA
SNGGGKQALETVQRLLPVLCQAHGLTPEQVVAIASNGGGKQALETVQRLLPVLCQA
HGLTPAQVVAIASNNGGKQALETVQRLLPVLCQDHGLTPAQVVAIASNIGGKQALETV
QRLLPVLCQAHGLTPEQVVAIASNGGGKQALETVQRLLPVLCQAHGLTPDQVVAIAS
NNGGKQALETVQRLLPVLCQAHGLTPAQVVAIASHDGGKQALETVQRLLPVLCQDH
GLTPDQVVAIASNGGGKQALETVQRLLPVLCQAHGLTPDQVVAIASHDGGKQALETV
QRLLPVLCQAHGLTPDQVVAIASNIGGKQALETVQRLLPVLCQAHGLTPAQVVAIASH
DGGKQALETVQRLLPVLCQAHGLTPAQVVAIASHDGGKQALETVQRLLPVLCQDHG
LTPAQVVAIASHDGGKQALETVQRLLPVLCQAHGLTPEQVVAIASNGGGKQALESIVA
QLSRPDPALAALLVGSAIPVKRGATGETKVFTGNSNSPKSPTKGGCSGGSTNLSDIIEK
ETGKQLVIQESILMLPEEVEEVIGNKPESDILVHTAYDESTDENVMLLTSDAPEYKPWA
LVIQDSNGENKIKML (SEQ ID NO: 10)
Left sTALED 
MALSRAVCGTSRQLAPVLGYLGSRQKHSLPDYPYDVPDYAGYPYDVPDYAG
YPYDVPDYAGIRIQDLRTLGYSQQQEKIKPKVRSTVAQHHEALVGHGFTHAHIVALS
QHPAALGTVAVKYQDMIAALPEATHEAIVGVGKQWSGARALEALLTVAGELRGPPLQ
LDTGQLLKIAKRGGVTAVEAVHAWRNALTGAPLNLTPDQVVAIASHDGGKQALETVQ
RLLPVLCQAHGLTPDQVVAIASNGGGKQALETVQRLLPVLCQDHGLTPAQVVAIASNI
GGKQALETVQRLLPVLCQDHGLTPDQVVAIASNNGGKQALETVQRLLPVLCQAHGL
TPDQVVAIASHDGGKQALETVQRLLPVLCQDHGLTPDQVVAIASHDGGKQALETVQR
LLPVLCQAHGLTPAQVVAIASNGGGKQALETVQRLLPVLCQDHGLTPAQVVAIASNIG
GKQALETVQRLLPVLCQAHGLTPDQVVAIASNNGGKQALETVQRLLPVLCQAHGLTP
 
AQVVAIASHDGGKQALETVQRLLPVLCQDHGLTPDQVVAIASHDGGKQALETVQRLL
PVLCQAHGLTPEQVVAIASNNGGKQALETVQRLLPVLCQAHGLTPAQVVAIASNGGG
KQALETVQRLLPVLCQAHGLTPAQVVAIASNGGGKQALETVQRLLPVLCQAHGLTPE
QVVAIASNGGGKQALESIVAQLSRPDPALAALTNDHLVALACLGGRPALDAVKKGLLV
GSAIPVKRGATGETKVFTGNSNSPKSPTKGGCSGSETPGTSESATPESSEVEFSHEYWM
RHALTLAKRARDEREVPVGAVLVLNNRVIGEGWNRAIGLHDPTAHAEIMALRQGGLV
MQNYRLIDATLYVTFEPCVMCAGAMIHSRIGRVVFGVRNSKRGAAGSLMNVLNYPG
MNHRVEITEGILADECAALLCDFYRMPRQVFNAQKKAQSSIN (SEQ ID NO: 11)
Right sTALED:

MASVLTPLLLRGLTGSARRLPVPRAKIHSLDYKDHDGDYKDHDIDYKDDDD
KGIRIQDLRTLGYSQQQQEKIKPKVRSTVAQHHEALVGHGFTHAHIVALSQHPAALGT
VAVKYQDMIAALPEATHEAIVGVGKQWSGARALEALLTVAGELRGPPLQLDTGQLLK
IAKRGGVTAVEAVHAWRNALTGAPLNLTPDQVVAIASNNGGKQALETVQRLLPVLCQ
DHGLTPAQVVAIASNIGGKQALETVQRLLPVLCQAHGLTPEQVVAIASNNGGKQALET
VQRLLPVLCQAHGLTPDQVVAIASNGGGKQALETVQRLLPVLCQAHGLTPAQVVAIA
SNGGGKQALETVQRLLPVLCQAHGLTPEQVVAIASNGGGKQALETVQRLLPVLCQA
HGLTPAQVVAIASNNGGKQALETVQRLLPVLCQDHGLTPAQVVAIASNIGGKQALETV
QRLLPVLCQAHGLTPEQVVAIASNGGGKQALETVQRLLPVLCQAHGLTPDQVVAIAS
NNGGKQALETVQRLLPVLCQAHGLTPAQVVAIASHDGGKQALETVQRLLPVLCQDH
GLTPDQVVAIASNGGGKQALETVQRLLPVLCQAHGLTPDQVVAIASHDGGKQALETV
QRLLPVLCQAHGLTPDQVVAIASNIGGKQALETVQRLLPVLCQAHGLTPAQVVAIASH
DGGKQALETVQRLLPVLCQAHGLTPAQVVAIASHDGGKQALETVQRLLPVLCQDHG
LTPAQVVAIASHDGGKQALETVQRLLPVLCQAHGLTPEQVVAIASNGGGKQALESIVA

QLSRPDPALAALTNDHLVALACLGGRPALDAVKKGLGGSGSGSYALGPYQISAPQLPA

YNGQTVGTFYYVNDAGGLESKVFSSGGPTPYPNYANAGHVEGQSALFMRDNGISEG

LVFHNNPEGTCGFCVNMTETLLPENAKMTVVPPEG (SEQ ID NO: 12)

### Example 3: Sequencing for base editing confirmation and measurement of base-editing efficiency

The reactant obtained in Example 2 was used as a template without purification and was subjected to targeted deep sequencing to analyze the sequence (The Sanger sequencing technique can alternatively be used for sequencing.) In this step, the template was amplified using a DNA polymerase with error tolerance to uracil or hypoxanthine (inosine as a nucleoside). The obtained base-editing results are shown in FIGS. 1 and 2. The base-editing results shown in FIG. 1 were obtained using the left and right DdCBE base editors, and base-editing results shown in FIG. 1 and FIG. 2 using the left and right sTALED as base editors.

### Example 4: Intracellular base-editing

HEK293T cells were maintained at 37°C in 5% CO₂ and cultured in TC-Treated Multiple Well Plates (Corning) using DMEM (Welgene) supplemented with 10% fetal bovine serum (Welgene) and 1% antibiotic-antimycotic solution. HEK293T cells were seeded at 0.75 × 10⁵ cells per well in a 48-well plate, and 0.5 µg of a plasmid comprising DNA encoding the left DdCBE (or sTALED) and 0.5 µg of a plasmid comprising DNA encoding the right DdCBE were mixed, followed by the addition of 25 µl of Opti-MEM (mixture 1). Then, 1.5 µl of lipofectamine 2000 and 25 µl of Opti-MEM were mixed (mixture 2). Mixture 1 and mixture 2 were mixed in a volume ratio of 1:1 and incubated at room temperature for 15 minutes. The cultured mixture was added at 50 µl per well, cultured for 4 days, and then the cell culture medium was removed. Cell lysis was performed by adding 100 µl of a cell lysis buffer (50 mM Tris, 1 mM EDTA, 0.05% SDS, and Proteinase K in a volume ratio of 1:20) per well. Thereafter, it was incubated at 50°C for 1 hour and 80°C for 20 minutes, and then stored at 4°C. After amplification using deep-sequencing primers that may distinguish only mitochondrial DNA, it was sequenced. The obtained base-editing results were compared with the base-editing results obtained through the cell-free test method in Example 3 (FIGS. 3 and 4).

As confirmed in FIG. 3, all base sequences that were C-to-T edited by DdCBE through the cell-free test method were also edited through the cellular experiment, and all base sequences that were A-to-G edited by TALED were likewise edited through the cellular experiment. In addition, for example, in the ACTCAATCCT CTGATC sequence, C-to-T base-editing occurred at a relatively high frequency in the underlined base sequence through the cell-free test method, and in the ACTCAATCCT CTGATC sequence, A-to-G base editing (referring to the case in which the A base paired with the underlined T base was edited) also occurred at a relatively high frequency, and this tendency of base-editing was likewise confirmed through the cellular experiment.

### Example 5: Amplification of template DNA for correction of m.A3460G

To select a base editor that corrects the G3460A mutation in the ND1 gene, which is the cause of LHON, a representative mitochondrial genetic disease, a double-stranded DNA sequence comprising the G3460A mutation was synthesized as a gBlock DNA fragment from IDT (Integrated DNA Technologies). The sequence of the obtained template DNA is as follows.

CTTCAAGGACGACGGCAACCTAGGCTATATACAACTACGCAAAGGCCCCA ACGTTGTAGGCCCCTACGGGCTACTACAACCCTTCGCTGACACCATAAAACTCTTC ACCAAAGAGCCCCTAAAACCCGCCACATCTACCATCACCCTCTACATCACCGCCCC GACCTTAGCGGCATCAAGGTGAACTTCA (The underlined sequence is an arbitrary PCR amplified sequence used to prevent contamination; and the bold A indicates the G3460A mutation.)

Template DNA was amplified by PCR using the forward primer (CTTCAAGGACGACGGCAACCTAGGCTATATACAACTACGC) and reverse primer (TGAAGTTCACCTTGATGCCGCTAAGGTCGGGGCGGTGATG), and then purified using a PCR SV mini kit (Geneall). Immediately before the experiment, the DNA was freshly diluted in distilled water to a concentration of 10 ng/µl and used.

### Example 6: Creation of a base editor for correction of m.A3460G

TALED comprising the T7 promoter (TAATACGACTCACTATAG) was produced. Specifically, TAL effector array plasmids and expression plasmids were constructed using the Golden-Gate cloning system. Competent DH5α (enzynomics) Escherichia coli cells were transformed by the heat shock (42°C) method, and single colonies were cultured in LB medium by shaking incubation overnight at 37°C. Plasmid DNA was purified using the Plasmid SV mini kit (GeneAll) according to the manufacturer's protocol. The base sequence of the purified plasmid DNA, that is, TALEDs, was confirmed by Sanger sequencing (Macrogen).

The amino acid sequences of the produced base-editing proteins are as follows.
17 C: 
MASVLTPLLLRGLTGSARRLPVPRAKIHSLDYKDHDGDYKDHDIDYKDDDD
KGIRIQDLRTLGYSQQQQEKIKPKVRSTVAQHHEALVGHGFTHAHIVALSQHPAALGT
VAVKYQDMIAALPEATHEAIVGVGKQWSGARALEALLTVAGELRGPPLQLDTGQLLK
IAKRGGVTAVEAVHAWRNALTGAPLNLTPDQVVAIASNIGGKQALETVQRLLPVLCQ
DHGLTPDQVVAIASHDGGKQALETVQRLLPVLCQAHGLTPAQVVAIASNNGGKQALE
TVQRLLPVLCQDHGLTPEQVVAIASNNGGKQALETVQRLLPVLCQAHGLTPAQVVAI
ASNNGGKQALETVQRLLPVLCQDHGLTPDQVVAIASHDGGKQALETVQRLLPVLCQ
DHGLTPEQVVAIASNGGGKQALETVQRLLPVLCQAHGLTPDQVVAIASNIGGKQALET
VQRLLPVLCQAHGLTPAQVVAIASHDGGKQALETVQRLLPVLCQAHGLTPEQVVAIA
SNGGGKQALETVQRLLPVLCQAHGLTPDQVVAIASNIGGKQALETVQRLLPVLCQAH
GLTPAQVVAIASHDGGKQALETVQRLLPVLCQDHGLTPDQVVAIASNIGGKQALETV
QRLLPVLCQDHGLTPAQVVAIASNIGGKQALETVQRLLPVLCQAHGLTPAQVVAIASH
DGGKQALETVQRLLPVLCQAHGLTPEQVVAIASNGGGKQALESIVAQLSRPDPALAAL
TNDHLVALACLGGRPALDAVKKGLLVGSAIPVKRGATGETKVFTGNSNSPKSPTKGG
CSGSETPGTSESATPESSEVEFSHEYWMRHALTLAKRARDEREVPVGAVLVLNNRVIG
EGWNRAIGLHDPTAHAEIMALRQGGLVMQNYRLIDATLYVTFEPCVMCAGAMIHSRI
GRVVFGVRNSKRGAAGSLMNVLNYPGMNHRVEITEGILADECAALLCDFYRMPRQV
FNAQKKAQSSIN (SEQ ID NO: 13)
18C: 
MASVLTPLLLRGLTGSARRLPVPRAKIHSLDYKDHGDYKDHDIDY
KDDDDKGIRIQDLRTLGYSQQQQEKIKPKVRSTVAQHHEALVGHGFTAHHIVALSQHP
 
AALGTVAVKVQDMIAALPEATHEAIVGVGKQWSGARALLEALLTVAGELRGPPLQLD
TGQLLKiAKRGGVTAVEAVHAWRNALTGAPLNLTPDQVVAIASNIGGKQALETVQRL
LPVLCQDHGLTPDQVVAIASHDGGKQALETVQRLLPVLCQAHGLTPAQVVAIASNNG
GKQALETVQRLLPVLCQDHGLTPEQVVAIASNNGGKQALETVQRLLPVLCQAHGLTP
AQVVAIASNNGGKQALETVQRLLPVLCQDHGLTPDQVVAIASHDGGKQALETVQRLL
PVLCQAHGLTPAQVVAIASHDGGKQALETVQRLLPVLCQAHGLTPEQVVAIASNGGG
KQALETVQRLLPVLCQAHGLTPAQVVAIASHDGGKQALETVQRLLPVLCQAHGLTPA
QVVAIASHDGGKQALETVQRLLPVLCQDHGLTPAQVVAIASNIGGKQALETVQRLLP
VLCQAHGLTPAQVVAIASHDGGKQALETVQRLLPVLCQDHGLTPAQVVAIASHDGGK
QALETVQRLLPVLCQAHGLTPEQVVAIASNGGGKQALESIVAQLSRPDPALAALTNDH
LVALACLGGRPALDAVKKGLLVGSAIPVKRRGATGETKVFTGNSNSPKSPTKGGCSGS
ETPGTSESATPESSEVEFSHEYWMRHALTLAKRARDEREVPVGAVLVLNNRVIGEGW
NRAIGLHDPTAHAEIMALRQGGLVMQNYRLIDATLYVTFEPCVMCAGAMiHSRIGRV
VFGVRNSKRGAAGSLMNVLNYPGMNHRVEITEGILADECAALLCDFYRMPQVFNAQ
KKAQSSIN(SEQ ID NO: 14)
56 N: 
MALSRAVCGTSRQLAPVLGYLGSRQKHSLPDYPYDVPDYAGYPYDVPDYAG
YPYDVPDYAGIRIQDLRTLGYSQQQEKIKPKVRSTVAQHHEALVGHGFTHAHIVALS
QHPAALGTVAVKYQDMIAALPEATHEAIVGVGKQWSGARALEALLTVAGELRGPPLQ
LDTGQLLKIAKRGGVTAVEAVHAWRNALTGAPLNLTPAQVVAIASHDGGKQALETVQ
RLLPVLCQAHGLTPDQVVAIASNGGGKQALETVQRLLPVLCQAHGLTPAQVVAIASN
GGGKQALETVQRLLPVLCQDHGLTPEQVVAIASNGGGKQALETVQRLLPVLCQAHG
LTPAQVVAIASNNGGKQALETVQRLLPVLCQDHGLTPDQVVAIASNNGGKQALETVQ
 
RLLPVLCQDHGLTPAQVVAIASNGGGKQALETVQRLLPVLCQAHGLTPAQVVAIASN
NGGKQALETVQRLLPVLCQDHGLTPAQVVAIASNIGGKQALETVQRLLPVLCQAHGL
TPDQVVAIASNIGGKQALETVQRLLPVLCQDHGLTPAQVVAIASNNGGKQALETVQR
LLPVLCQDHGLTPAQVVAIASNIGGKQALETVQRLLPVLCQAHGLTPDQVVAIASNNG
GKQALETVQRLLPVLCQAHGLTPAQVVAIASNGGGKQALETVQRLLPVLCQAHGLTP
EQVVAIASNGGGKQALESIVAQLSRPDPALAALTNDHLVALACLGGRPALDAVKKGLG
GSGSGSYALGPYQISAPQLPAYNGQTVGTFYYVNDAGGLESKVFSSGGPTPYPNYAN
AGHVEGQSALFMRDNGISEGLVFHNNPEGTCGFCVNMTETLLPENAKMTVVPPEG
(SEQ ID NO: 15)
57 N:

MALSRAVCGTSRQLAPVLGYLGSRQKHSLPDYPYDVPDYAGYPYDVPDYAG
YPYDVPDYAGIRIQDLRTLGYSQQQEKIKPKVRSTVAQHHEALVGHGFTHAHIVALS
QHPAALGTVAVKYQDMIAALPEATHEAIVGVGKQWSGARALEALLTVAGELRGPPLQ
LDTGQLLKIAKRGGVTAVEAVHAWRNALTGAPLNLTPAQVVAIASHDGGKQALETVQ
RLLPVLCQAHGLTPDQVVAIASNGGGKQALETVQRLLPVLCQAHGLTPAQVVAIASN
GGGKQALETVQRLLPVLCQDHGLTPEQVVAIASNGGGKQALETVQRLLPVLCQAHG
LTPAQVVAIASNNGGKQALETVQRLLPVLCQDHGLTPDQVVAIASNNGGKQALETVQ
RLLPVLCQDHGLTPAQVVAIASNGGGKQALETVQRLLPVLCQAHGLTPAQVVAIASN
NGGKQALETVQRLLPVLCQDHGLTPAQVVAIASNIGGKQALETVQRLLPVLCQAHGL
TPDQVVAIASNIGGKQALETVQRLLPVLCQDHGLTPAQVVAIASNNGGKQALETVQR
LLPVLCQDHGLTPAQVVAIASNIGGKQALETVQRLLPVLCQAHGLTPDQVVAIASNNG
GKQALETVQRLLPVLCQAHGLTPDQVVAIASNGGGKQALETVQRLLPVLCQAHGLTP
AQVVAIASNGGGKQALETVQRLLPVLCQAHGLTPEQVVAIASNGGGKQALESIVAQL

SRPDPALAALTNDHLVALACLGGRPALDAVKKGLGGSGSGSYALGPYQISAPQLPAYN
GQTVGTFYYVNDAGGLESKVFSSGGPTPYPNYANAGHVEGQSALFMRDNGISEGLVF
HNNPEGTCGFCVNMTETLLPENAKMTVVPPEG (SEQ ID NO: 16)

### Example 7: In vitro transcription and translation (IVTT) and base editing for correction of m.A3460G

After quantifying the template DNA obtained in Example 5 to a concentration of 10 ng/µL, a reaction mixture (with distilled water added up to 25 µL), which contained 10 ng of the template DNA, 0.5 µg of a plasmid comprising DNA encoding a base-editing protein having an amino acid sequence of SEQ ID NO: 13 or 14, 0.5 µg of a plasmid comprising DNA encoding a base-editing protein having an amino acid sequence of SEQ ID NO: 15 or 16, and 20 µL of an in vitro coupled transcription/translation (IVTT) kit mixture, was mixed in a tube, incubated at 30°C for 6 hours, and then incubated at 37°C for 16 hours. The IVTT kit used was TNT^{®} Quick Coupled Transcription/Translation Systems (Promega), and the TNT^{®} Quick Master Mix, comprising TNT^{®} Rabbit Reticulocyte Lysate, TNT^{®} Reaction Buffer, TNT^{®} RNA Polymerase, Amino Acid Mixture, and RNasin^{®} Ribonuclease Inhibitor, was used.

The obtained base-editing result is shown in FIG. 5.

### Example 8: Intracellular base-editing for correction of m.A3460G

Primary cells were isolated from the urine of a LHON patient carrying the G3460A point mutation in the mitochondrial ND1 gene, subdivided at passage 2, and cryopreserved. Urine-derived cells (UDCs) from the patient were cultured at 37°C in 5% CO₂ in 12-well Clear TC-Treated Multiple Well Plates (Corning) coated with 0.1% gelatin (Welgene), using Renal Epithelial Cell Growth Medium Bullet Kit (REGM, Lonza). The UDC cells were seeded in 96-well Clear TC-Treated Multiple Well Plates (Corning) at a density of 0.6 × 10⁴ cells per well. After 20-24 hours, 250 ng of a plasmid encoding base-editing protein 17C or 18C and 250 ng of a plasmid encoding base-editing protein 56N or 57N were transfected using Lipofectamine LTX reagent (Invitrogen) and introduced into the pre-seeded 96-well plate. The transfected cells were cultured at 37°C in 5% CO₂ while replacing the culture medium.

The transfected UDC cells were lysed by adding 100 µL per well of a cell lysis buffer (50 mM Tris-HCl pH 7.4 (Welgene), 1 mM EDTA pH 8.0 (Welgene), 0.005% sodium dodecyl sulfate (Welgene), and 1:20 volume ratio of Proteinase K (Qiagen)), were incubated in a PCR machine at 50°C for 1 hour and 80°C for 20 minutes; and were stored at 4°C.

The cell lysis was directly used as a template without purification, and the sequence was analyzed using a targeted deep sequencing technique to assess the base-editing ratio in the target site. To construct a deep sequencing library, nested first and second PCRs were performed using PrimeSTAR^{®} GXL DNA Polymerase (TAKARA) and deep-sequencing primers capable of selectively amplifying mitochondrial DNA, and then a third PCR was performed using an index-containing primer to add the final index sequence. The third PCR product with index sequences was purified using the PCR SV mini kit (GeneAll), and paired-end sequencing was performed using the MiniSeq Mid Output Kit (Illumina) with the MiniSeq system (Illumina).

The obtained base-editing result is shown in FIG. 6 and was compared with the base-editing result obtained through the cell-free test method of Example 7 (FIGS. 7 to 10). As confirmed in FIG. 10, all base editors that showed on-target editing ability for the G3460A mutation in the cell-free test also demonstrated the same on-target editing ability in the intracellular experiments. In addition, as shown in FIGS. 7 to 9, it was also confirmed that not only the on-target editing ability but also the bystander (non-target) base-editing efficiency exhibited a similar tendency between the cell-free and intracellular test methods.

## Claims

1. A method for providing information on the ability of a base editing system to correct a DNA mutation, the method comprising:
(a) contacting an isolated DNA comprising a target DNA mutation with one or more base-editing proteins used in a base-editing system in a cell-free environment;
(b) sequencing the DNA obtained from step (a) to measure the base-editing activity of the base-editing system; and
(c) providing information on the ability of the base-editing system to correct the same DNA mutation in a cell.

2. The method according to claim 1, wherein step (a) involves mixing an isolated DNA comprising a target DNA mutation with one or more polynucleotides encoding one or more base-editing proteins, and wherein the one or more polynucleotides are transcribed and translated in a cell-free environment.

3. The method according to claim 2, wherein the one or more polynucleotides encoding the one or more base-editing proteins are DNA or mRNA.

4. The method according to any one of claims 1 to 3, wherein, in step (a), a base-editing protein is expressed and a DNA base is edited through a one-pot reaction.

5. The method according to claim 1, wherein step (a) involves mixing an isolated DNA comprising a target DNA mutation with a base-editing protein in the cell-free environment.

6. The method according to any one of claims 1 to 5, wherein a reaction product of step (a) is used in step (b) without separate purification.

7. The method according to any one of claims 1 to 6, wherein the target DNA mutation comprises one or more single nucleotide mutations.

8. The method according to any one of claims 1 to 7, wherein the isolated DNA comprising the target DNA mutation is extracted and amplified from an animal or plant cell, or is artificially synthesized.

9. The method according to any one of claims 1 to 8, wherein the isolated DNA comprising the target DNA mutation is nuclear DNA or organelle DNA.

10. The method according to claim 9, wherein the organelle DNA is mitochondrial DNA or chloroplast DNA.

11. The method according to any one of claims 1 to 10, wherein the isolated DNA comprising the target DNA mutation is nuclear DNA or mitochondrial DNA from a human having a genetic disease.

12. The method according to any one of claims 1 to 11, wherein the base editing is performed to convert an adenine (A) base to guanine (G), a cytosine (C) base to thymine (T), or both.

13. The method according to any one of claims 1 to 12, wherein the one or more base-editing proteins each independently comprise one or more DNA binding proteins selected from the group consisting of a zinc finger protein, a transcriptional activator-like effector (TALE) protein, a CRISPR-associated nuclease, and a CRISPR-associated nickase.

14. The method according to claim 13, wherein the one or more DNA binding proteins comprise a CRISPR-associated nuclease, and in step (a), the guide RNA is further contacted with the isolated DNA comprising the target DNA mutation.

15. The method according to claim 13, wherein the one or more DNA binding proteins comprise a CRISPR-associated nickase, and in step (a), a reverse transcriptase or a polynucleotide encoding the reverse transcriptase, and a guide RNA are further contacted with the isolated DNA comprising the target DNA mutation.

16. The method according to any one of claims 1 to 15, wherein one or more of the base proteins each independently comprise a base-converting enzyme.

17. The method according to claim 16, wherein the one or more base-converting enzymes independently comprise one or more of APOBEC (apolipoprotein B editing complex), AID (activation-induced deaminase), TadA (tRNA-specific adenosine deaminase), or DddA tox, or a variant thereof.

18. The method according to claim 17, wherein DddA tox or a variant thereof is used in the form of two splits.

19. The method according to claim 17, wherein DddA tox or a variant thereof is used in the form of a full-length protein.

20. The method according to any one of claims 1 to 19, wherein the one or more base-editing proteins each independently comprise a uracil glycosylase inhibitor (UGI).

21. A method for screening a base editor capable of correcting a target DNA mutation in cells, the method comprising:
(a) a step of contacting an isolated DNA comprising a target DNA mutation with candidate base editors in a cell-free environment, wherein the candidate base editors each independently comprise one or more base-editing proteins or one or more polynucleotides encoding the same;
(b) measuring the base editing activity of the candidate base editors by sequencing the DNA molecules obtained from step (a);
(c) providing information on the ability of the candidate base editors to correct the same DNA mutation in cells; and
(d) selecting a base editor capable of correcting the same DNA mutation in cells based on the information obtained from step (c).

22. A base editor selected by the method according to claim 21.

23. A kit for use in a method according to any one of claims 1 to 21, comprising a DNA polymerase, an RNA polymerase, a mixture of ribonucleotide triphosphates, a mixture of amino acids, a ribosome comprising tRNA and rRNA, an initiation factor, an elongation factor, a termination factor, and a buffer.
